# EUROPEAN PATENT SPECIFICATION

(11) **EP 1 242 009 B1**
(45) Date of publication and mention of the grant of the patent: **22.08.2007**
(21) Application number: 00984404.4
(22) Date of filing: 15.12.2000
(51) Int. Cl.: A61F 5/00, A61K 9/00, A61M 35/00

(54) **Tissue sensitizing compounds for females with methods and apparatus for the delivery of these compounds**
Zusammensetzungen zur Gewebesenisibilisierung weiblicher Personen und Verfahren und Vorrichtung zur Verabreichung dieser Verbindungen
Compositions stimulant les tissus de femmes et procédés et appareils pour leur application

(30) Priority: 21.12.1999 US 469959; 07.03.2000 US 520110
(43) Date of publication of application: 25.09.2002
(73) Proprietor: 40 J'S, LLC, Ft. Thomas, KY 41075 (US)
(72) Inventor: THOMPSON, Ronald, J., Ft. Thomas, KY 41075 (US)
(74) Representative: Torggler, Paul Norbert
(86) International application number: PCT/US2000/033987
(87) International publication number: WO 2001/045599

(56) References cited:
- WO-A-99/13717
- WO-A-99/20266
- US-A- 4 139 006
- US-A- 5 386 836
- US-A- 5 460 597

## Description

### Background of the Invention

### Field of the Invention

This invention relates to arrangements for the topical application of stimulants comprising menthol and L-arginine to the lips or the clitoris of human females.

### Prior Art

The unique properties of the clitoral sensitizing compounds encompasses the vasodilatation of clitoral blood vessels by the initial effect of menthol to facilitate and promote the absorption of L-arginine when topically applied to the mucous membrane of the clitoris. The L-arginine stimulates the nitric oxide synthase mediated production of nitric oxide to effect clitoral sensitivity, arousal, and erection by sustained vasodilatation. Both of these actions are specific to the topical application of the compound to the mucous membrane of the clitoris and presume an inert, non-active base or vehicle. In the menthol/L-arginine composition, the menthol actually acts as a vehicle to enhance the absorption of L-arginine.

In the human female, decreased clitoral sensitivity and responsiveness are related to normal aging, relative or absolute estrogen and testosterone deficiency (either as a consequence of medicines or aging), and by a host of vascular conditions such as diabetes and hypertension. Multiple laboratory and clinical research endeavors have been directed toward male erectile dysfunction (ED), yielding not only an understanding of the erection physiology, but also medications to treat ED, such as Viagra^{™}. Very little research has been initiated to understand or address female physiological sexual unresponsiveness. However, since the penis and the clitoris are analogous anatomical structures, the basic cellular and physiological knowledge about male penile erections translates to functions of the clitoris.

Female clitoral dysfunction is extremely difficult to document and quantify. A number of modalities, such as Doppler blood flow, precise temperature measurements, and actual imaging measurements, have been employed to attempt to define clitoral responsiveness - all with results unsatisfactory for meaningful research. Estimates that 15 million U.S. men suffer from erectile dysfunction have been reported in the literature. A recent article in the February 10, 1999 issue of the *Journal of the American Medical Association* suggests that female erectile dysfunction occurs probably twice the rate of male ED, therefore affecting 30 million U.S. women.

In the female anatomy, the clitoral artery is located in the middle of the clitoris, extending lengthwise from the base to the tip of the clitoris, and supplies blood to the clitoris. Two clitoral veins are located on either side of the clitoral artery, and normally drain the clitoris of the blood pumped into it from the artery. As female sexual arousal initiates, either by direct stimulation of the clitoris or by the application of one of the recently developed female arousal creams, the vessels dilate and the valves of the clitoral veins located at the base of the clitoris close. The venous blood fills two honeycomb-like chambers, the corpus cavernosa. The corpus cavernosa are normally empty of blood, but, like the clitoral artery and veins, they are positioned lengthwise from the base to the tip of the clitoris. Therefore, as the valves in the veins at the base of the clitoris close, the blood pumped into the clitoris artery distends the corpus cavernosa. This causes the clitoris to enlarge two-to-three fold, and to become erect, rigid, and highly sensitive, just as the penis in the male. In fact, the penis and the clitoris are the exact same structures. Female clitoral enlargement and rigidity and male penile erection are both accomplished by the same action: vasodilation of the vascular structures maximally distending the corpus cavernosa.

Topical application of medications to different types of Integument (skin) to effect changes in sensation are based upon that type of integument. There are two types of integument (skin): keratinized stratified squamous epithelium, and non-keratinized stratified squamous epithelium, more commonly referred to as mucous membrane. The entire external surface of the body is covered with keratinized stratified squamous epithelium except the lips, mouth, anus, and the vagina/vulva in females. If a lotion is applied to the keratinized squamous epithelium, it is absorbed only by the top layers of the skin. Multiple transdermal medications are delivered through the keratinized skin, but the delivery system is very sophisticated. Absorption of medications is much easier if they are delivered to mucous membranes, or non-keratinized stratified squamous epithelium, especially the lips, the vulva and vaginal mucosa. Many vaginal creams and suppositories that dissolve to become creams with the moisture and heat of the vagina have been sold for over fifty years. All of the vaginal creams and suppositories are absorbed into full thickness of the non-keratinized stratified squamous epithelium that defines the vaginal mucosa. Some of the vaginal creams are systemically absorbed by the blood vessels in the basement membrane of the vaginal mucosa, while other medications are not systemically absorbed. Systemic absorption refers to the distribution of the medication throughout all tissues of the body via the blood stream. The vaginal/vulvar mucosa is a multiple layer of non-keratinized stratified squamous epithelial cells twenty-to-thirty cells in thickness from the basement membrane to the outermost cells of the mucosa.

The integument of the undercarriage of the clitoris is non-keratinized stratified squamous epithelium (mucous membrane), and topical medications are readily absorbed. The clitoral hood that covers the dorsal aspect of the clitoris is partially keratinized, and allows partial absorption of topical medications. The integument of the penis is keratinized stratified squamous epithelium. Absorption of the same medication topically applied to the penis and the clitoris concurrently will be much more readily absorbed by the clitoris because of the mucous membrane of the clitoris.

Topical menthol is a particular tissue or clitoral sensitizing agent and may be applied to clitoral tissue and may have a number of effects. The specific effects that menthol causes are dependent upon three variables: the concentration of the menthol in the preparation, the vehicle and its evaporation properties, and most importantly, the type of tissue to which the menthol is applied. If the menthol is in a non-evaporating vehicle and applied to keratinized stratified squamous epithelium (hairy skin), a low concentration is under 1%, a mid-range concentration, 1%-5%, and a high concentration, between 5% and 10%. If menthol in a non-evaporating vehicle is applied to non-keratinized stratified squamous epithelium (mucous membrane), a low concentration is less than 0.5%, a mid-range concentration between 0.5% and 2%, and a high concentration between 2% and 5%. Therefore, a 1% concentration of menthol in a non-evaporating vehicle applied to keratinized and non-keratinized epithelium would produce very different effects: the keratinized epithelium would respond with a minimal effect, while the non-keratinized epithelium would respond with a marked effect. This is because of the rapid absorption through the non-keratinized epithelium (mucous membrane) and the relatively poor absorption through the barrier-like keratinized epithelium (hairy skin).

Menthol is highly lipid-soluble and readily absorbed by a mucous membrane. A mid-range concentration of 0.5% to 2% of menthol topically applied to mucous membrane causes almost immediate smooth muscle relaxation, hyperemia, and vasodilation of blood vessels by a direct response. Menthol also causes the immediate stimulation of both thermoreceptors and nociceptors. The thermoreceptors, when stimulated, create a sensation of warmth and burning for this menthol concentration in mucous membrane (like the clitoris). The nociceptors initiate an axon reflex to release vasodilator peptides. The vasodilator peptides cause an immediate vasodilation of blood vessels with resultant blood engorgement and transudate production. The sera component of the blood that leaks out of the blood vessels to provide increased vaginal/vulvar lubrication is due to the nociceptor initiated vasodilation. The onset of all of these mechanisms occurs within thirty seconds of application of the menthol preparation, and these responses persist for fifteen-to-twenty minutes. Therefore, the topical application of a 1% menthol preparation, in a non-evaporating vehicle directly to the mucous membrane of the clitoris, causes the following physiological responses: a. warmth, tingling, and even mild burning; b. hyperemia; c. direct smooth muscle relaxation; d. indirect blood engorgement via nociceptor-mediated vasodilation by peptide release; and e. increased vulvar/vaginal lubrication.

Topical L-arginine is a clitoral sensitizing agent, produced from intracellular nitric acid (NO), which is a potent vasodilator of smooth muscles, and has been identified and confirmed by a number of research studies to be pivotally involved in penile/clitoral erections. Nitric oxide is synthesized from the substrate L-arginine in a cellular reaction catalyzed by nitric oxide synthase (NOS). Two different types of NOS have been identified: eNOS and nNOS. The eNOS is contained with the smooth muscle endothelial cells that line the vasculature of the penis/clitoris. As the available L-arginine is concerted into NO by eNOS, the blood vessels and corpus cavernosa of the penis/clitoris dilate and fill with blood. This engorgement of the penis/clitoris is the mechanism for the erection and rigidity of the aroused penis/clitoris. The nNOS is contained within the nerve cells of the penis/clitoris and converts L-arginine into NO to sensitize the penis/clitoris and cause the release of vasodilation peptide. Burnett, et al. reported in "Immunohistochemical Description of Nitric Oxide Synthase Isoforms in Human Clitoris", Journal of Urology, July 1997, 75-79, that the vascular and neuronal alteration of the penis/clitoris that create an erection are the direct effect of the ability of the cells to convert the available L-arginine into nitric oxide through nitric oxide synthase.

Several urologic and gynecologic reports have identified a correlation between a decreased availability or activity of the NO synthase system and normal aging, testosterone deficiency, and estrogen deficiency. The studies confirm that an increase in the supply of the substrate L-arginine allows the attenuated but still functional NOS system to affect the nitric oxide medicated erection of the penis/clitoris. Therefore, the topical application of L-arginine via the NOS system causes vasodilation of the penile/clitoral veinous structures and concomitant sensitivity. The safety of 1% of 2% menthol topically applied to tissue such as the clitoris is a consideration. Two different strengths of menthol may be commercialized, such as 1% and 2% concentrations in a non-evaporating water base. The anticipated normal volume of such cream to be applied to the clitoris is 300 mg. Therefore, the maximum amount of menthol delivered is 6 mg. *The Extra Pharmacopoeia,* Martindale, 1989, reports menthol toxicity for humans at 28 mg./kg. In a 50 kg individual, the toxic dose could be 1400 mg., but this is estimated to be extremely low by Eccles in his "Review: Menthol and Other Cooling Compounds" in the 1994 Journal of Pharmacological Pharmacopedia, volume 46, pages 618-630. A 6 mg. dose of menthol is well below the 1400 mg. dose calculated and gives a 100 plus fold margin of safety.

The safety of L-arginine topically applied to tissue such as the clitoris is also a consideration. Two different strengths of L-arginine USP, 2% and 4%, may be marketed so as to sensitize the clitoris. The anticipated normal volume of such cream to be applied to the clitoris is 300 mg. Therefore, a maximum of 12 mg of L-arginine USP is available. If all 12 mg. are systemically absorbed, this is a small fraction of what has been reported in the gynaecologic literature as safe. Fracchinette, et al., reported the intravenous infusion of 30 grams (30,000 milligrams) of L-arginine into 29 pregnant patients without adverse effects in the 1999 Journal of the Society of Gynecologic Investigation, volume 6,202-207.

WO 99/13717 discloses a delivery vehicle and method for delivering, either topically or orally, a nitric oxide releasing substance such as L-arginine into human or mammalian tissue for the purpose of producing beneficial effects such as the relief of pain, the warming of cold tissues, the growth of hair on the scalp, the healing of leg ulcers, the relief of impotence as well as other beneficial effects. The delivery vehicle provides a hostile biophysical environment which facilitates and promotes the migration of the nitric oxide releasing substance into the tissue.

WO 99/20266 discloses a method for enhancing female sexual desire and response, comprising topically administering to the clitoris of a subject in need thereof an effective amount of a prostaglandin.

### Brief Summary of the Invention

The present invention also relates to alternate preparations of comprising L-Arginine and menthol as well as related cooling compounds that comprises a class of single-source of botanical or essential oils that can be used individually or as a combination of several oils such as: Peppermint oil; Commint oil; Eucalyptus oil; Citronella oil; Indian turpentine oil; Camphor oil and Cinnamon oil.

In fact, all of the botanicals listed by Steinberg in "Frequency Use of Botanicals, " in Cosmetic and Toiletries magazine, Volume 113, October, 1998, are members of this class of single-source botanical or essential oils. Potentially, any of these referenced oils could evoke the menthol-like effect on the mucous membrane to facilitate or promote the topical absorption of L-arginine. In addition, known minor skin irritants can cause a profound reaction when topically applied to mucous membrane, such as redness, irritation, and reflex vasodilatation. This irritant reaction associated with vasodilatation shares some similarities with the menthol effect, and could quite effectively substitute for the menthol in promoting L-arginine absorption and actions. Salicylate and capsiatin are two of the commonly use minor skin irritants. Oil soluble vitamins (coenzymes) A, D, or E, could also Potentiate absorption of menthol, L-arginine, minor skin irritants, or any of the menthol-related cooling compounds. The oil soluble vitamins could be used to substitute for, or be used in addition to, any of the previously listed components in a topical clitoral sensitizing preparation.

The invention may further comprise alternate preparations for the base or the vehicle. Such biologically active agents (menthol or its substitutes) and L-arginine can be compounded in a non-biologically active base, or in a biologically active base that promotes absorption, a vehicle. Any base or vehicle is intended to liquefy at body heat and in the presence of moisture present in mucous membrane when topically applied to mucous membrane tissue. Campos and Eccleston is "Vitamin A Skin Penetration", "Cosmetics and Toiletries magazine, volume 113, July, 1998, describe and quantify how different vehicles influence and promote the hairy skin (keratinized stratified squamous epithelium) absorption of Vitamin A. Mucous membranes absorb solutions more readily than hairy skin, but display linear absorption potentials relative to the vehicles studies by Campos and Eccleston. An active vehicle may be engineered that synergistically functions to promote the absorption and actions of menthol, menthol-related compounds, biologically derived oils, minor skin irritants, oil soluble vitamins, L-arginine, or any combination of these.

There are solid/liquid state dynamics for the topical delivery of clitoral sensitizing compounds which cover any of the potential compounds for topical application to sensitize tissues such as the clitoris because they can have different solid/liquid states at ambient and at body temperatures. A solid compound, such as exemplified by the Chap Stick® Lip Balm, A. H. Robbins Company, of Richmond, Virginia, could be directly applied to the undercarriage of the clitoris, and liquefy at body temperature and in the moisture inherent in mucous membrane tissue. Liquefied compounds are readily absorbed, dependent on various other factors described. A gel/cream or liquid compound could be directly applied to the clitoris for topical absorption. Like the solid-state compound, the gel/cream must liquefy before absorption can be effected. The dynamics of how rapidly a compound transforms from a solid state or gel/cream state to a liquid state could be controlled to evoke an almost immediate effect, or a relatively delayed effect, before absorption of the compound.

A crystalline related dissolution may be different from the temperature related dissolution of a solid or a gel/cream. Small crystals of menthol, L-arginine, or any of the previously described components, may be suspended in a base vehicle. Their availability for absorption would depend on their dissolution from a crystalline state to a liquid state. The crystalline effect may be designed to control the rate of absorption: for instance, whether the compound were available for absorption immediately on application, or if a delayed, sustained absorption over a period of time were desired. Both of these parameters could allow the discrete, private application of the clitoral-sensitizing compound in anticipation of intercourse, without knowledge of the partner.

Because the clitoral-sensitizing compounds are also intended to function independently without the preferred intercourse-related physical stimulation of the clitoris, the menthol/L-arginine compounds may also be arranged to be available in various strength to address the needs of all women. The menthol may be compounded in multiple strengths, ranging from 0.1 % to 5%, and any increment in between. The L-arginine may also be compounded in multiple strengths ranging from 1% to 10%. The spectrum of different strengths may be compounded into a single delivery system, such as lip balm, or alternatively, in all of the potential delivery systems: solid, gel/cream, and liquid.

A single use or a multiple use delivery systems comprising clitoral-sensitizing preparations may be individually packaged within a small tube or packet for single use. Conversely, a multiple-dose reusable delivery system, like a tube of hand cream or toothpaste, or a stick of lip balm, may be packaged for personal use.

The apparatus and methods to deliver a topical preparation to the lips or the clitoris may be comprised of a solid or semi-solid compound that may be directly applied to the undercarriage of the clitoris regardless of the use of a single-or multiple-use delivery system. The clitoral contact area of the solid or semi-solid compound in one preferred embodiment will have a notch to increase the surface area and clitoral contact of the compound. The notch is arranged with a height of 0.5 to 2 centimeters, and a maximum width of 2 centimeters at the rim of the notch. A properly designed concave notch will topically apply the compound to the 180 degree undercarriage of the clitoris. With proper directions for use, the application surface will initially contact the vestibular tissue at the base of the clitoris, to spread the compound on the entire clitoris.

A gel/cream or liquid compound would be applied to the tissue or clitoris with the same motions, but would require a different type of application device. The gel/cream or liquid could be applied by "roller balls" like those used to apply viscous deodorants, by a sponge-type applicator, or by a brush type of system. Any of the delivery devices for gel/cream or liquids could be designed to increase the surface area of the applicator tip and generally use the concave notch described for the solid compounds. The gel/cream and liquid applicators would have either a single-use reservoir or a multi-use reservoir. All of the delivery devices would have a cap that would seal the device before the initial use, and protect the applicator tip from contamination before use. Multi-use devices would have a resealing cap.

The invention thus comprises a hand-manipulable, tissue or clitoral stimulant-applying applicator for use to apply a clitoral stimulating compound to the clitoral area of the human female, comprising: a reservoir for containment of a clitoral stimulating compound; a clitoral stimulating compound in said reservoir; and a removable cover on the reservoir to expose the compound to permit said compound to be applied topically. The cover may comprise a removable cap. The reservoir may comprise a tube and said cover. The compound may comprise a gel. The compound may comprise a cream. The compound may comprise a fluid liquid. The compound may comprise a semi-solid. The semi-solid compound may have a notch on its distalmost end to facilitate the application of the compound. The applicator may include a brush thereon. The applicator may include a sponge thereon. The applicator may include a roller ball arrangement thereon. The compound may be comprised of a mixture of menthol and L-arginine. The compound may have components selected from the group consisting of: Peppermint oil, Cornmint oil, Eucalyptus oil, Citronella oil, Indian turpentine oil, Camphor oil and Cinnamon oils. The compound may have components selected from the group consisting of: Salicylate, capsiatin, and oil soluble vitamins (co-enzymes) of A, D, or E.

The invention may also include a method of sensitizing the tissue or clitoris of a human female, comprising the steps of: applying a compound of sensitizing agent to the tissue or clitoris; and selecting a component of said sensitizing agent from the group consisting of: Peppermint oil, Commint oil, Eucalyptus oil, Citronella oil, Indian turpentine oil, Camphor oil, Cinnamon oils, Salicylate, capsiatin, and oil soluble vitamins (co-enzymes) of A, D, or E. The steps may include placing the sensitizing agent in a reservoir of an applicator; arranging a cover on the applicator to protect the agent in the reservoir. The applicator may include a brush. The applicator may include a roller at one end thereof. The applicator may include a sponge at one end thereof. The sensitizing agent comprises a semi-solid stick having a "V" notch on a distal end thereof to facilitate application of the agent to the tissue such as a clitoris.

Further unique properties of a combination menthol/L-arginine topical clitoral sensitizing preparation provides the basis of the present invention. Sequential application first by menthol applied topically to the mucous membrane of the clitoris has an almost immediate response by affecting vasodilation and blood engorgement warmth (via the thermoreceptors). The duration of menthol's action on mucous membranes may be for fifteen-to-twenty minutes. Subsequent topical application of L-arginine utilizes its topical or systemic effect, via the NOS synthase/NO system, to create corpus cavernosa engorgement, which has a delayed onset of action from between fifteen to twenty minutes. By combining the two compounds, with simultaneous topical application of menthol and L-arginine, their effect would be immediate and prolonged because of their two different, but related, methods of action.

L-arginine absorption is promoted by the actions of menthol. Eccles reports studies by Katayama (1972), Morimoto (1993) and Yamo (1991) where the topical application of menthol, because of its local vasodilatative and lipidphillic properties, promotes the penetration of other topical drug preparation applied concurrently with menthol. These studies found rapid absorption of indomethacin, cortisone, morphine hydrochloride, and methyl salicylate when applied topically in combination with menthol. It is only rational that the absorption of L-arginine by the mucous membrane is hastened when applied in combination with menthol. This is a direct action of the lipidphillic and vasodilation properties of menthol. The action of menthol causes the L-arginine NOS/NO vasodilation to be effective within minutes of the topical application of the menthol/L-arginine combination.

A different effect is realized on the clitoris and the penis using the same menthol/L-arginine combination. This is because the integument of the clitoris is non-keratinized epithelium, a 1% menthol and 2% L-arginine preparation, topically applied, is immediately absorbed, and immediately active in sensitizing the clitoris. The penis is relatively unaffected by the application of the same strength cream because the external epithelium is keratinized squamous epithelium. The keratinized squamous epithelium, by nature of the keratin, acts as a barrier to the absorption of the preparation. Therefore, the same preparation of 1% menthol and 2% L-arginine combination sensitizes the clitoris, without sensitizing the penis.

The invention thus comprises a topical compound preparation of sensitizing cream for application to the non-keratinized epithelium of a female, comprising a preparation of L-arginine, and a preparation of menthol. The preparation of L-arginine may have a concentration of 4% or less. The preparation of menthol may have a concentration of 5% or less. The preparation of L-arginine may in one embodiment have a concentration of 2% and the preparation of menthol may have a concentration of 1%.

The compound preparation may in one embodiment be enclosed within a reservoir of an applicator. The reservoir may have multiple chambers. The reservoir may have an arrangement of conduits from the reservoir to an outer clitoral-touching surface on the applicator. The invention also includes a method of sensitizing the non-keratinized epithelium of a female, comprising the steps of: applying a preparation of menthol and L-arginine to the epithelium, arranging the preparation of L-arginine in a concentration of less than 4%; and arranging the preparation of menthol in a concentration of less than 5%. The method may include the step of: placing the preparations of menthol and L-arginine in an applicator reservoir for distribution onto the epithelium, and may include the step of applying the preparations to the epithelium in a sequential manner.

### Brief Description of the Drawings

Figure 1 is a front elevational view of a solid compound single-use applicator;
Figure 2 is a view taken along the lines 2-2 of Figure 1;
Figure 3 is a front elevational view of a solid compound multiple-use applicator;
Figure 4 is a view taken along the lines 4-4 of Figure 3;
Figure 5 is a front elevational view of a gel/cream/liquid compound single-use applicator;
Figure 6 is a front elevational view of a gel/cream/liquid compound single or multiple use brush applicator;
Figure 7 is a view of the applicator shown in Figure 6, with its cap off;
Figure 8 is a side elevational view, in section, of the applicator cap shown in Figure 6;
Figure 9 is front elevational view of a gel/cream or liquid applicator with roller balls;
Figure 10 is a view taken along the lines 10-10 of Figure 9; and

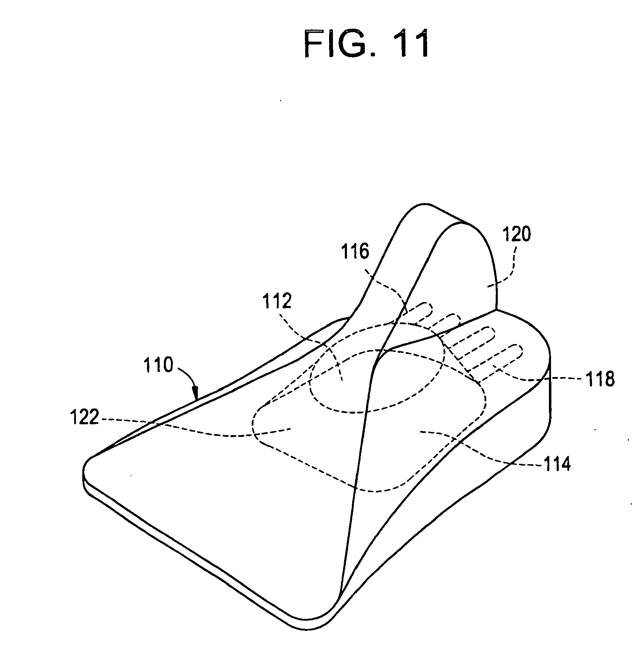

Figure 11 is a perspective view of a further applicator of the present invention.

### Description of Preferred Embodiments

The compositions may additionally comprise a class of single-source of botanical or essential oils that can be used individually or as a combination of several oils such as: Peppermint oil; Cornmint oil; Eucalyptus oil; Citronella oil; Indian turpentine oil; Camphor oil and Cinnamon oils, as compounds of the present invention. Any of these referenced oils could evoke the menthol-like effect on the mucous membrane to facilitate or promote the topical absorption of L-arginine. Salicylate and capsiatin, two commonly used minor skin irritants and oil soluble vitamins (co-enzymes) A, D, or E, could also potentiate absorption of menthol, L-arginine, minor skin irritants, or any of the menthol-related cooling compounds. The oil soluble vitamins could be used to substitute for, or be used in addition to, any of the previously listed components in a topical tissue or clitoral sensitizing preparation. Such solid/liquid state dynamics for the topical delivery of tissue or clitoral sensitizing compounds which cover any of the potential compounds for topical application to sensitize the tissue of lips or the clitoris because they can have different solid/liquid states at ambient and at body temperatures.

A gel/cream or liquid compound could be directly applied to the tissue or clitoris for topical absorption. Like the solid-state compound, the gel/cream must liquefy before absorption can be effected. The dynamics of how rapidly a compound transforms from a solid state or gel/cream state to a liquid state could be controlled to evoke an almost immediate effect, or a relatively delayed effect, before absorption of the compound.

A crystalline related dissolution may be different from the temperature related dissolution of a solid or a gel/cream. Small crystals of menthol, L-arginine, or any of the previously described components, may be suspended in a base vehicle. Their availability for absorption would depend on their dissolution from a crystalline state to a liquid state. The crystalline effect may be designed to control the rate of absorption: for instance, whether the compound were available for absorption immediately on application, or if a delayed, sustained absorption over a period of time were desired. Both of these parameters could allow the discrete, private application of the clitoral-sensitizing compound in anticipation of intercourse, without knowledge of the partner.

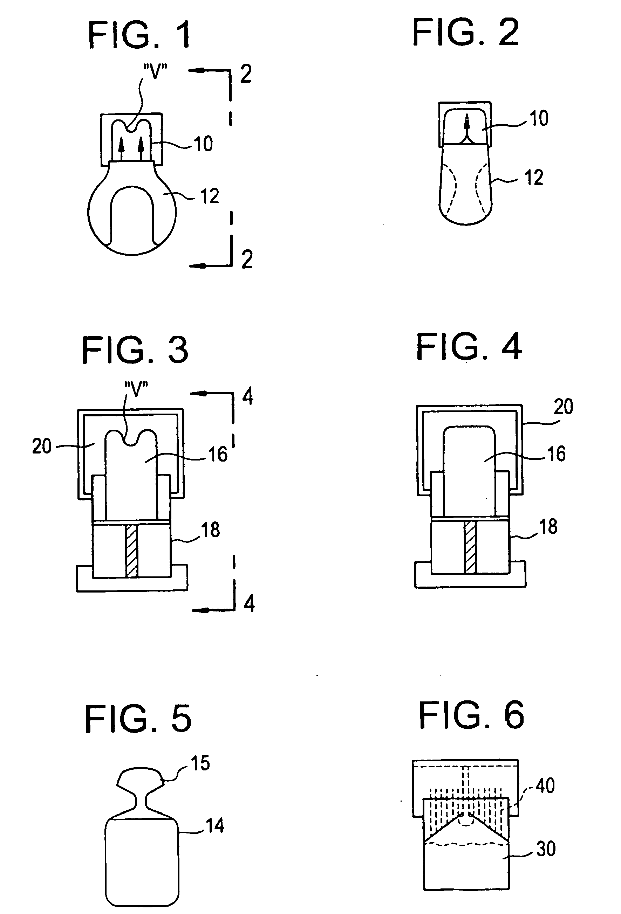

A single use or a multiple use delivery systems comprising a tissue or clitoral-sensitizing preparation 10 may be individually packaged within a small tube 12 or packet 14 for single use, as shown in Figures 1, 2 and 5. The tube 12 may be gripped by the thumb and first finger, and the compound preparation thereon applied as desired.

The packet 14 shown in Figure 5 has a break-away seal 15 which permits the gel/cream or liquid 10 therein to be readily applied by merely squeezing the packet 14. Conversely, a multiple-dose reusable delivery system, like a tube of hand cream or toothpaste, or a stick of semi-solid compound, tissue such as lips or clitoral-sensitizing balm 16, may be packaged for personal use, as shown by the "lipstick-like" rotatively adjustable applicators 18 in Figures 3 and 4. This adjustable applicator 18 has a cap 20 to cover the balm compound 16.

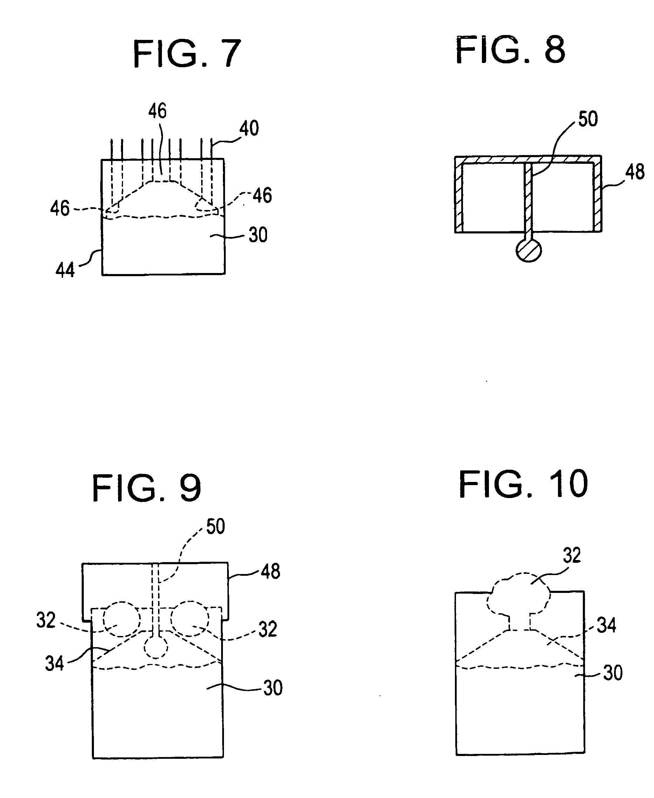

The apparatus and methods to deliver a topical preparation to the tissue such as lips or clitoris may also be comprised of a solid or semi-solid compound that may be directly applied to the lips of the mouth or the undercarriage of the clitoris regardless of the use of a single-or multiple-use delivery system. The clitoral contact area of the solid or semi-solid compound in one preferred embodiment will have a notch "V" to increase the surface area and clitoral contact of the compound 16, as shown in Figures 1 and 3. The notch "V" is arranged with a height of 0.5 to 2 centimeters, and a maximum width of 2 centimeters at the rim of the notch "V". A properly designed concave notch "V" will topically apply the compound 16 to the 180 degree undercarriage of the clitoris. With proper directions for use, the application surface will initially contact the vestibular tissue at the base of the clitoris, to spread the compound on the entire clitoris. A gel/cream or liquid compound 30 of the present invention would be applied to the clitoris with the same motions, but would require a different type of application device. The gel/cream or liquid compound 30 may be applied by "roller balls" 32 like those used to apply viscous deodorants, as shown in Figures 9 and 10, supported within a foraminous top 34, covered when not needed, by a cap 46. The gel/cream or liquid compound 30 may also be applied by a sponge or brush 40 secured to a top 42 in a reservoir 44, as shown in Figures 6 and 7. A cap 48, shown also in Figure 8 may include a removable seal 50, both of which are arranged to be removable to expose the sponge or brush 40 and openings 46 in the reservoir 44 to supply the fluid compound thereto. Any of the delivery devices for gel/cream or liquids could be designed to increase the surface area of the applicator tip and generally use the concave notch described for the solid compounds. The gel/cream and liquid applicators would have either a single-use reservoir or a multi-use reservoir. All of the delivery devices would have a cap "C" that would seal the device before the initial use, and protect the applicator tip from contamination before use. Multi-use devices, such as shown in Figures 3, 4, 6, 7 and 9 would have the resealing cap 48 for their protection.

The present invention also utilizes the unique properties of a combination menthol/L-arginine topical clitoral sensitizing preparation. In one embodiment, a sequential application first by menthol applied topically to the mucous membrane of the clitoris has an almost immediate response by affecting vasodilation and blood engorgement warmth (via the thermoreceptors). The duration of menthol's action on mucous membranes may be for fifteen-to-twenty minutes. Subsequent topical application of L-arginine utilizes its topical or systemic effect, via the NOS synthase/NO system, to create corpus cavernosa engorgement, which has a delayed onset of action from between fifteen to twenty minutes. Such a sequential delivery of multiple preparations may be accomplished by an applicator 110, as shown in Figure 10. The applicator 110 may have a plurality of reservoirs 112 and 114, each with their own conduits 116 and 118 between the reservoirs 112 and 114 and a clitoral engaging surface 120. Each preparation of menthol and L-arginine may have their own rate of distribution, to provide the sequential application to the treatment situs.

In a further embodiment, a common reservoir 122 may be arranged within the applicator 110. By combining the two compounds within that common reservoir 122 as a simultaneous topical application of menthol and L-arginine together, their effect would be immediate and prolonged because of their two different, but related, methods of action. Such a range of L-arginine of less than 4% concentration and a range of menthol of less than 5% concentration is preferred in an application, either with the distribution via an applicator 110, or by other topical application.

## Claims

1. A hand-manipulable, tissue stimulant-applying applicator for use to apply a tissue stimulating composition to the non-keratinized clitoral or lip tissue of a human comprising:
a reservoir for containment of a tissue stimulating composition;
a tissue stimulating composition comprising a mixture of menthol and L-arginine disposed in said reservoir; and
an opening on said reservoir to permit exposure of said composition to permit said composition to be applied topically.

2. The hand-manipulable, tissue stimulant-applying applicator of claim 1, further comprising a removable cap to cover the opening.

3. The hand-manipulable, tissue stimulant-applying applicator of claim: 1, wherein said reservoir comprises a tube and said opening.

4. The hand-manipulable, tissue stimulant-applying applicator of claim 1, wherein said composition comprises a gel.

5. The hand-manipulable, tissue stimulant-applying applicator of claim 1, wherein said composition comprises a cream.

6. The hand-manipulable, tissue stimulant-applying applicator of claim 1, wherein said composition comprises a fluid liquid.

7. The hand-manipulable, tissue stimulant-applying applicator of claim 1, wherein said composition comprises a semi-solid.

8. The hand-manipulable, tissue stimulant-applying applicator of claim 7, wherein said semi-solid composition has a notch on its distal most end to facilitate the application of said composition.

9. The hand-manipulable, tissue stimulant-applying applicator of claim 6, wherein said applicator includes a brush thereon.

10. The hand-manipulable, tissue stimulant-applying applicator of claim 6, wherein said applicator includes a sponge thereon.

11. The hand-manipulable, tissue stimulant-applying applicator of claim 6, wherein said applicator includes a roller ball arrangement thereon.

12. The hand-manipulable, tissue stimulant-applying applicator of claim 1, wherein said composition has components selected from the group consisting of Peppermint oil, Commint oil, Eucalyptus oil, Citronella oil, Indian turpentine oil, Camphor oil and Cinnamon oils.

13. The hand-manipulable, tissue stimulant-applying applicator of claim 1, wherein said composition has components selected from the group consisting of Salicylate, capsiatin, and oil soluble vitamins of A, D, or E.

14. The hand-manipulable, tissue stimulant-applying applicator of claim 1 wherein the sensitive area of non-keratinized tissue are the lips of the mouth.

15. The hand-manipulable, tissue stimulant-applying applicator of claim 1 wherein the sensitive area of non-keratinized tissue is the vagina of a human female.

16. The hand-manipulable, tissue stimulant-applying applicator of claim 1 wherein the sensitive area of non-keratinized tissue is the anus of a human

17. The hand-manipulable, tissue stimulant-applying applicator of claim 1 wherein said opening of said reservoir is created by a removable portion of the reservoir.

18. A topical composition preparation of sensitizing cream for application to the non-keratinized epithelium of a human comprising:
a preparation of L-arginine;
a preparation of menthol.

19. The topical composition preparation of claim 18, wherein said preparation of L-arginine has a concentration of 10% or less.

20. The topical composition preparation of claim 18, wherein said preparation of menthol has a concentration of 5% or less.

21. The topical composition preparation of claim 18, wherein said preparation of L-arginine has a concentration of 2% and said preparation of menthol has a concentration of 1%.

22. The topical composition preparation of claim 18, wherein said composition preparation is enclosed within a reservoir of an applicator.

23. The topical composition preparation of claim 22, wherein said reservoir has multiple chambers.

24. The topical composition preparation of claim 22, wherein the external non-keratinized epithelium of a female is the clitoral portion of the vagina of a female and, wherein said reservoir has an arrangement of conduits from said reservoir to an outer clitoral touching surface on said applicator.

25. The topical composition preparation of claim 24, wherein said preparation of L-arginine is in a concentration of less than 4% and wherein said preparation of menthol is in a concentration of less than 5%.

26. The hand-manipulable applicator of claim 1 further comprising a single use removable portion to create the opening in the reservoir to permit exposure of said composition and to permit said composition to be applied topically.

27. The hand manipulable applicator of claim 26 wherein said composition is a gel.

28. The hand manipulable applicator of claim 26 wherein said composition is a semi-solid.

29. The hand manipulable applicator of claim 26 wherein said composition is a cream.

30. The hand manipulable applicator of claim 26 wherein said composition is a liquid.

31. The hand manipulable applicator of claim 26 wherein said composition is a sponge.

32. The hand manipulable applicator of claim 26 wherein said composition is a brush.

33. The hand manipulable applicator of claim 26 wherein said composition is a roller ball.

34. The hand manipulable applicator of claim 26, wherein the composition further comprises a compound selected from the group consisting of peppermint oil, cornmint oil, eucalyptus oil, citronella oil, Indian turpentine oil, camphor oil, vitamin A, vitamin D, vitamin E, salicylate, capsiatin, and cinnamon oil.

35. The hand manipulable applicator of claim 26 wherein the concentration of menthol in the composition is less than 5%.

36. The hand manipulable applicator of claim 26 wherein the concentration of menthol in the composition is less than 4%.

37. The hand manipulable applicator of claim 26 wherein the concentration of L-arginine in the composition is less than 4%.

38. The hand manipulable applicator of claim 26 wherein the concentration of L-arginine in the composition is less than 2%.

39. The hand manipulable applicator of claim 26 wherein the concentration of L-arginine in the composition is 1%.

40. A method for the preparation of a composition for applying to the non-keratinized clitoral or lip tissue of a human comprising admixing menthol and L-arginine in a carrier to form a composition.

41. The method of claim 40 further comprising the step of:
admixing a composition selected from the group consisting of peppermint oil, cornmint oil, eucalyptus oil, citronella oil, Indian turpentine oil, camphor oil, vitamin A, vitamin D, vitamin E, salicylate, capsiatin, and cinnamon oil with the L-arginine and menthol.

42. The method of claim 40, wherein the composition comprises less than 4% L-arginine.

43. The method of claim 40, wherein the composition comprises less than 2% L-arginine.

44. The method of claim 40 wherein the composition comprises less than 5% menthol.

45. A topical composition preparation of sensitizing cream for the treatment of female erectile dysfunction comprising:
a preparation of L-arginine; and,
a preparation of menthol.

46. The topical composition of claim 45 wherein the L-arginine has a concentration of less than 10% and the menthol has a concentration of less than 5%.

47. The topical composition of claim 46 further comprising:
a composition selected from the group consisting of peppermint oil, cornmint oil, eucalyptus oil, citronella oil, Indian turpentine oil, camphor oil, vitamin A, vitamin D, vitamin E, salicylate, capsiatin, cinnamon oil, and combinations thereof.

48. The method of preparing a topical composition preparation of sensitizing cream for the treatment of female erectile dysfunction comprising the steps of:
admixing a preparation of L-arginine and a preparation of menthol.

49. The method of claim 48 further comprising the step of:
admixing a composition selected from the group consisting of peppermint oil, cornmint oil, eucalyptus oil, citronella oil, Indian turpentine oil, camphor oil, vitamin A, vitamin D, vitamin E, salicylate, capsiatin, and cinnamon oil with L-arginine preparation and the menthol preparation.

50. The method of claim 48 further comprising the step of:
adjusting the composition wherein the L-arginine has a concentration of less than 10% and the menthol has a concentration of less than 5%.

## Patentansprüche

1. Ein von Hand betätigbarer, ein Gewebe-Stimulans auftragender Applikator zur Verwendung, um eine gewebestimulierende Zusammensetzung auf das nicht-keratinisierte klitorale oder Lippengewebe eines Menschen aufzutragen, umfassend:
Einen Behälter zur Aufnahme einer gewebestimulierenden Zusammensetzung;
eine gewebestimulierende Zusammensetzung, die eine Mischung aus Menthol und L-Arginin aufweist und in dem genannten Behälter angeordnet ist; und
eine Öffnung in dem genannten Behälter, um den Austritt der genannten Zusammensetzung zu erlauben, um eine topische Anwendung der genannten Zusammensetzung zu erlauben.

2. Der von Hand betätigbare, ein Gewebe-Stimulans auftragende Applikator aus Anspruch 1, weiters umfassend eine abnehmbare Kappe zur Abdeckung der Öffnung.

3. Der von Hand betätigbare, ein Gewebe-Stimulans auftragende Applikator aus Anspruch 1, wobei der genannte Behälter ein Rohr und die genannte Öffnung aufweist.

4. Der von Hand betätigbare, ein Gewebe-Stimulans auftragende Applikator aus Anspruch 1, wobei die genannte Zusammensetzung ein Gel umfasst.

5. Der von Hand betätigbare, ein Gewebe-Stimulans auftragende Applikator aus Anspruch 1, wobei die genannte Zusammensetzung eine Creme umfasst.

6. Der von Hand betätigbare, ein Gewebe-Stimulans auftragende Applikator aus Anspruch 1, wobei die genannte Zusammensetzung eine Flüssigkeit umfasst.

7. Der von Hand betätigbare, ein Gewebe-Stimulans auftragende Applikator aus Anspruch 1, wobei die genannte Zusammensetzung eine halbfeste Zusammensetzung ist.

8. Der von Hand betätigbare, ein Gewebe-Stimulans auftragende Applikator aus Anspruch 7, wobei die genannte halbfeste Zusammensetzung eine Einkerbung auf ihrem entferntesten Ende aufweist, um das Auftragen der genannten Zusammensetzung zu vereinfachen.

9. Der von Hand betätigbare, ein Gewebe-Stimulans auftragende Applikator aus Anspruch 6, wobei der genannte Applikator eine Bürste darauf umfasst.

10. Der von Hand betätigbare, ein Gewebe-Stimulans auftragende Applikator aus Anspruch 6, wobei der genannte Applikator einen Schwamm darauf umfasst.

11. Der von Hand betätigbare, ein Gewebe-Stimulans auftragende Applikator aus Anspruch 6, wobei der genannte Applikator eine Rollkugel-Anordnung darauf umfasst.

12. Der von Hand betätigbare, ein Gewebe-Stimulans auftragende Applikator aus Anspruch 1, wobei die genannte Zusammensetzung Bestandteile enthält, die ausgewählt wurden aus der Gruppe bestehend aus Pfefferminzöl, Ackerminzöl, Eukalyptusöl, Zitronengrasöl, indischem Terpentinöl, Kampferöl und Zimtöle.

13. Der von Hand betätigbare, ein Gewebe-Stimulans auftragende Applikator aus Anspruch 1, wobei die genannte Zusammensetzung Bestandteile enthält, die ausgewählt wurden aus der Gruppe bestehend aus Salicylat, Capsiatin und der löslichen Vitamine A, D oder E.

14. Der von Hand betätigbare, ein Gewebe-Stimulans auftragende Applikator aus Anspruch 1, wobei der sensible Bereich des nicht-keratinisierten Gewebes die Lippen des Mundes sind.

15. Der von Hand betätigbare, ein Gewebe-Stimulans auftragende Applikator aus Anspruch 1, wobei der sensible Bereich des nicht-keratinisierten Gewebes die Vagina einer menschlichen Frau ist.

16. Der von Hand betätigbare, ein Gewebe-Stimulans auftragende Applikator aus Anspruch 1, wobei der sensible Bereich des nicht-keratinisierten Gewebes der Anus eines Menschen ist.

17. Der von Hand betätigbare, ein Gewebe-Stimulans auftragende Applikator aus Anspruch 1, wobei die genannte Öffnung des genannten Behälters von einem abnehmbaren Teil des Behälters gebildet wird.

18. Die Zubereitung einer topischen Zusammensetzung einer sensibilisierenden Creme zur Anwendung auf dem nicht-keratinisierten Epithel eines Menschen umfassend:
Eine Zubereitung L-Arginin;
eine Zubereitung Menthol.

19. Die Zubereitung einer topischen Zusammensetzung aus Anspruch 18, wobei die genannte L-Arginin-Zubereitung eine Konzentration von 10 % oder weniger aufweist.

20. Die Zubereitung einer topischen Zusammensetzung aus Anspruch 18, wobei die genannte Menthol-Zubereitung eine Konzentration von 5 % oder weniger aufweist.

21. Die Zubereitung einer topischen Zusammensetzung aus Anspruch 18, wobei die genannte L-Arginin-Zubereitung eine Konzentration von 2 % und die genannte Menthol-Zubereitung eine Konzentration von 1 % aufweist.

22. Die Zubereitung einer topischen Zusammensetzung aus Anspruch 18, wobei die genannte Zubereitung der Zusammensetzung innerhalb eines Behälters eines Applikators eingeschlossen ist.

23. Die Zubereitung einer topischen Zusammensetzung aus Anspruch 22, wobei der genannte Behälter mehrere Kammern aufweist.

24. Die Zubereitung einer topischen Zusammensetzung aus Anspruch 22, wobei das äußerliche, nicht-keratinisierte Epithel einer Frau der klitorale Bereich der Vagina einer Frau ist und wobei der genannte Behälter eine Anordnung von Kanälen von dem genannten Behälter zu einer äußeren, klitoral anliegenden Fläche auf dem genannten Applikator aufweist.

25. Die Zubereitung einer topischen Zusammensetzung aus Anspruch 24, wobei die genannte L-Arginin-Zubereitung eine Konzentration von weniger als 4 % aufweist und wobei die genannte Menthol-Zubereitung eine Konzentration von weniger als 5 % aufweist.

26. Der von Hand betätigbare Applikator aus Anspruch 1, weiters umfassend ein abnehmbares Einwegteil, um die Öffnung im Behälter zu schaffen, um den Austritt der genannten Zusammensetzung zu erlauben und um eine topische Anwendung der genannten Zusammensetzung zu erlauben.

27. Der von Hand betätigbare Applikator aus Anspruch 26, wobei die genannte Zusammensetzung ein Gel ist.

28. Der von Hand betätigbare Applikator aus Anspruch 26, wobei die genannte Zusammensetzung eine halbfeste Substanz ist.

29. Der von Hand betätigbare Applikator aus Anspruch 26, wobei die genannte Zusammensetzung eine Creme ist.

30. Der von Hand betätigbare Applikator aus Anspruch 26, wobei die genannte Zusammensetzung eine Flüssigkeit ist.

31. Der von Hand betätigbare Applikator aus Anspruch 26, wobei die genannte Zusammensetzung ein Schwamm ist.

32. Der von Hand betätigbare Applikator aus Anspruch 26, wobei die genannte Zusammensetzung eine Bürste ist.

33. Der von Hand betätigbare Applikator aus Anspruch 26, wobei die genannte Zusammensetzung eine Rollkugel ist.

34. Der von Hand betätigbare Applikator aus Anspruch 26, wobei die genannte Zusammensetzung weiters eine Verbindung umfasst, die ausgewählt wurde aus der Gruppe bestehend aus Pfefferminzöl, Ackerminzöl, Eukalyptusöl, Zitronengrasöl, indischem Terpentinöl, Kampferöl, Vitamin A, Vitamin D, Vitamin E, Salicylat, Capsiatin und Zimtöl.

35. Der von Hand betätigbare Applikator aus Anspruch 26, wobei die Konzentration von Menthol in der Zusammensetzung weniger als 5 % beträgt.

36. Der von Hand betätigbare Applikator aus Anspruch 26, wobei die Konzentration von Menthol in der Zusammensetzung weniger als 4 % beträgt.

37. Der von Hand betätigbare Applikator aus Anspruch 26, wobei die Konzentration von L-Arginin in der Zusammensetzung weniger als 4 % beträgt.

38. Der von Hand betätigbare Applikator aus Anspruch 26, wobei die Konzentration von L-Arginin in der Zusammensetzung weniger als 2 % beträgt.

39. Der von Hand betätigbare Applikator aus Anspruch 26, wobei die Konzentration von L-Arginin in der Zusammensetzung weniger als 1 % beträgt.

40. Ein Verfahren für die Zubereitung einer Zusammensetzung zur Anwendung auf dem nicht-keratinisierten klitoralen oder Lippengewebe eines Menschen, das die Beimischung von Menthol und L-Arginin in einem Trägerstoff zur Bildung einer Zusammensetzung umfasst.

41. Das Verfahren aus Anspruch 40, das den Schritt umfasst:
Beimischung einer Zusammensetzung, die ausgewählt wurde aus der Gruppe bestehend aus Pfefferminzöl, Ackerminzöl, Eukalyptusöl,
Zitronengrasöl, indischem Terpentinöl, Kampferöl, Vitamin A, Vitamin D, Vitamin E, Salicylat, Capsiatin und Zimtöl zu dem L-Arginin und Menthol.

42. Das Verfahren aus Anspruch 40, wobei die Zusammensetzung weniger als 4 % L-Arginin aufweist.

43. Das Verfahren aus Anspruch 40, wobei die Zusammensetzung weniger als 2 % L-Arginin aufweist.

44. Das Verfahren aus Anspruch 40, wobei die Zusammensetzung weniger als 5 % Menthol aufweist.

45. Eine Zubereitung einer topischen Zusammensetzung einer sensibilisierenden Creme zur Behandlung von weiblicher erektiler Dysfunktion umfassend:
Eine L-Arginin-Zubereitung und
eine Menthol-Zubereitung.

46. Die topische Zusammensetzung aus Anspruch 45, wobei das L-Arginin eine Konzentration von weniger als 10 % aufweist und das Menthol eine Konzentration von weniger als 5 % aufweist.

47. Die topische Zusammensetzung aus Anspruch 46 weiters umfassend:
Eine Zusammensetzung ausgewählt aus der Gruppe bestehend aus Pfefferminzöl, Ackerminzöl, Eukalyptusöl, Zitronengrasöl, indischem Terpentinöl, Kampferöl, Vitamin A, Vitamin D, Vitamin E, Salicylat, Capsiatin, Zimtöl und Kombinationen daraus.

48. Das Verfahren der Zubereitung einer Zubereitung einer topischen Zusammensetzung einer sensibilisierenden Creme zur Behandlung von weiblicher erektiler Dysfunktion, das die Schritte umfasst:
Beimischung einer L-Arginin-Zubereitung und einer Menthol-Zubereitung.

49. Das Verfahren aus Anspruch 48, das weiters den Schritt umfasst:
Beimischung einer Zusammensetzung ausgewählt aus der Gruppe bestehend aus Pfefferminzöl, Ackerminzöl, Eukalyptusöl, Zitronengrasöl, indischem Terpentinöl, Kampferöl, Vitamin A, Vitamin D, Vitamin E, Salicylat, Kapsiatin und Zimtöl zu der L-Arginin-Zubereitung und der Menthol-Zubereitung.

50. Das Verfahren aus Anspruch 48, das weiters den Schritt umfasst:
Anpassen der Zusammensetzung, wobei das L-Arginin eine Konzentration von weniger als 10 % aufweist und das Menthol eine Konzentration von weniger als 5 % aufweist.

## Revendications

1. Dispositif applicateur pour l'application d'un stimulant tissulaire, manipulable manuellement, destiné à être utilisé pour appliquer une composition de stimulation tissulaire au tissu clitoridien ou labial non kératinisé d'un humain comprenant :
un réservoir pour contenir une composition de stimulation tissulaire ;
une composition de stimulation tissulaire comprenant un mélange de menthol et de L-arginine disposée dans ledit réservoir ; et
une ouverture sur ledit réservoir pour permettre l'exposition de ladite composition pour qu'elle soit appliquée de manière topique.

2. Dispositif applicateur pour l'application d'un stimulant tissulaire manipulable manuellement selon la revendication 1, comprenant en outre un couvercle retirable pour recouvrir l'ouverture.

3. Dispositif applicateur pour l'application d'un stimulant tissulaire manipulable manuellement selon la revendication 1, dans lequel ledit réservoir comprend un tube et ladite ouverture.

4. Dispositif applicateur pour l'application d'un stimulant tissulaire manipulable manuellement selon la revendication 1, dans lequel ladite composition comprend un gel.

5. Dispositif applicateur pour l'application d'un stimulant tissulaire manipulable manuellement selon la revendication 1, dans lequel ladite composition comprend une crème.

6. Dispositif applicateur pour l'application d'un stimulant tissulaire manipulable manuellement selon la revendication 1, dans lequel ladite composition comprend un fluide.

7. Dispositif applicateur pour l'application d'un stimulant tissulaire manipulable manuellement selon la revendication 1, dans lequel ladite composition comprend un semi-solide.

8. Dispositif applicateur pour l'application d'un stimulant tissulaire manipulable manuellement selon la revendication 7, dans lequel ladite composition semi-solide a une rainure sur son extrémité la plus distale pour faciliter l'application de ladite composition.

9. Dispositif applicateur pour l'application d'un stimulant tissulaire manipulable manuellement selon la revendication 6, pour lequel ledit dispositif applicateur comprend une brosse sur celui-ci.

10. Dispositif applicateur pour l'application d'un stimulant tissulaire manipulable manuellement selon la revendication 6, pour lequel ledit dispositif applicateur comprend une éponge sur celui-ci.

11. Dispositif applicateur pour l'application d'un stimulant tissulaire manipulable manuellement selon la revendication 6, pour lequel ledit dispositif applicateur comprend une disposition à bille roulante sur celui-ci.

12. Dispositif applicateur pour l'application d'un stimulant tissulaire manipulable manuellement selon la revendication 1, dans lequel ladite composition a des composants choisis dans le groupe constitué de l'huile de menthe poivrée, l'huile de menthe des champs, l'huile d'eucalyptus, l'huile de citronnelle, l'huile de térébenthine indienne, l'huile de camphre et l'huile de cannelle.

13. Dispositif applicateur pour l'application d'un stimulant tissulaire manipulable manuellement selon la revendication 1, dans lequel ladite composition a des composants choisis dans le groupe constitué de salicylate, capsiatine et vitamines A, B ou E solubles dans l'huile.

14. Dispositif applicateur pour l'application d'un stimulant tissulaire manipulable manuellement selon la revendication 1, dans laquelle la zone sensible de tissu non kératinisé concerne les lèvres de la bouche.

15. Dispositif applicateur pour l'application d'un stimulant tissulaire manipulable manuellement selon la revendication 1, dans laquelle la zone sensible de tissu non kératinisé est le vagin d'une femme humaine.

16. Dispositif applicateur pour l'application d'un stimulant tissulaire manipulable manuellement selon la revendication 1, dans laquelle la zone sensible de tissu non kératinisé est l'anus d'un être humain.

17. Dispositif applicateur pour l'application d'un stimulant tissulaire manipulable manuellement selon la revendication 1, dans lequel ladite ouverture dudit réservoir est créée par une partie retirable du réservoir.

18. Préparation de composition topique d'une crème de sensibilisation pour l'application à l'épithélium non kératinisé d'un être humain comprenant :
une préparation de L-arginine ;
une préparation de menthol.

19. Préparation de composition topique selon la revendication 18, dans laquelle ladite préparation de L-arginine a une concentration de 10% ou moins.

20. Préparation de composition topique selon la revendication 18, dans laquelle ladite préparation de menthol a une concentration de 5% ou moins.

21. Préparation de composition topique selon la revendication 18, dans laquelle ladite préparation de L-arginine a une concentration de 2% et ladite préparation de menthol a une concentration de 1%.

22. Préparation de composition topique selon la revendication 18, dans laquelle ladite préparation de la composition est enfermée dans un réservoir d'un dispositif applicateur.

23. Préparation de composition topique selon la revendication 22, dans laquelle ledit réservoir a des chambres multiples.

24. Préparation de composition topique selon la revendication 22, dans laquelle l'épithélium non kératinisé externe d'une femme est la partie clitoridienne du vagin d'une femme et, dans laquelle ledit réservoir a un dispositif de conduits depuis ledit réservoir vers une surface de toucher clitoridien externe sur ledit dispositif applicateur.

25. Préparation de composition topique selon la revendication 24, dans laquelle ladite préparation de L-arginine est à une concentration de moins de 4% et dans laquelle ladite préparation de menthol est à une concentration de moins de 5%.

26. Dispositif applicateur manipulable manuellement selon la revendication 1 comprenant en outre une partie retirable pour une utilisation unique pour créer l'ouverture dans le réservoir pour permettre l'exposition de ladite composition et permettre à ladite exposition d'être appliquée de manière topique.

27. Dispositif applicateur manipulable manuellement selon la revendication 26, dans lequel ladite composition est un gel.

28. Dispositif applicateur manipulable manuellement selon la revendication 26, dans lequel ladite composition est un semi-solide.

29. Dispositif applicateur manipulable manuellement selon la revendication 26, dans lequel ladite composition est une crème.

30. Dispositif applicateur manipulable manuellement selon la revendication 26, dans lequel ladite composition est un liquide.

31. Dispositif applicateur manipulable manuellement selon la revendication 26, dans lequel ladite composition est une éponge.

32. Dispositif applicateur manipulable manuellement selon la revendication 26, dans lequel ladite composition est une brosse.

33. Dispositif applicateur manipulable manuellement selon la revendication 26, dans lequel ladite composition est une bille roulante.

34. Dispositif applicateur manipulable manuellement selon la revendication 26, dans lequel la composition comprend en outre un composé choisi dans le groupe constitué de l'huile de menthe poivrée, l'huile de menthe des champs, l'huile d'eucalyptus, l'huile de citronnelle, l'huile de térébenthine indienne, l'huile de camphre, la vitamine A, la vitamine D, la vitamine E, le salicylate, le capsiatine et l'huile de cannelle.

35. Dispositif applicateur manipulable manuellement selon la revendication 26, dans lequel la concentration de menthol dans la composition est de moins de 5%.

36. Dispositif applicateur manipulable manuellement selon la revendication 26, dans lequel la concentration de menthol dans la composition est de moins de 4%.

37. Dispositif applicateur manipulable manuellement selon la revendication 26, dans lequel la concentration de L-arginine dans la composition est de moins de 4%.

38. Dispositif applicateur manipulable manuellement selon la revendication 26, dans lequel la concentration de L-arginine dans la composition est de moins de 2%.

39. Dispositif applicateur manipulable manuellement selon la revendication 26, dans lequel la concentration de L-arginine dans la composition est de 1%.

40. Procédé pour la préparation d'une composition pour l'application au tissu clitoridien ou labial non kératinisé d'un être humain comprenant le mélange du menthol et de la L-arginine dans un véhicule pour former une composition.

41. Procédé selon la revendication 40 comprenant en outre l'étape consistant à :
mélanger une composition choisie dans le groupe constitué de l'huile de menthe poivrée, l'huile de menthe des champs, l'huile d'eucalyptus, l'huile de citronnelle, l'huile de térébenthine indienne, l'huile de camphre, la vitamine A, la vitamine B, la vitamine E, le salicylate, le capsiatine et l'huile de cannelle avec la L-arginine et le menthol.

42. Procédé selon la revendication 40, dans lequel la composition comprend moins de 4% de L-arginine.

43. Procédé selon la revendication 40, dans lequel la composition comprend moins de 2% de L-arginine.

44. Procédé selon la revendication 40, dans lequel la composition comprend moins de 5% de menthol.

45. Préparation d'une composition topique d'une crème de sensibilisation pour le traitement d'un dysfonctionnement érectile féminin comprenant :
une préparation de L-arginine ; et
une préparation de menthol.

46. Composition topique selon la revendication 45, dans laquelle la L-arginine a une concentration de moins de 10% et le menthol a une concentration de moins de 5%.

47. Composition topique selon la revendication 46 comprenant en outre :
une composition choisie dans le groupe constitué de l'huile de menthe poivrée, l'huile de menthe des champs, l'huile d'eucalyptus, l'huile de citronnelle, l'huile de térébenthine indienne, l'huile de camphre, la vitamine A, la vitamine D, la vitamine E, le salicylate, le capsiatine, l'huile de cannelle et des combinaisons de ceux-ci.

48. Procédé de préparation d'une préparation d'une composition topique d'une crème de sensibilisation pour le traitement d'un dysfonctionnement érectile féminin comprenant l'étape consistant à :
mélanger une préparation de L-arginine et une préparation de menthol.

49. Procédé selon la revendication 48 comprenant en outre l'étape consistant à :
mélanger une composition choisie dans le groupe constitué de l'huile de menthe poivrée, l'huile de menthe des champs, l'huile d'eucalyptus, l'huile de citronnelle, l'huile de térébenthine indienne, l'huile de camphre, la vitamine A, la vitamine D, la vitamine E, le salicylate, le capsiatine, et l'huile de cannelle avec la préparation de L-arginine et la préparation de menthol.

50. Procédé selon la revendication 48, comprenant en outre l'étape consistant à :
ajuster la composition dans laquelle la L-arginine a une concentration de moins de 10% et le menthol a une concentration de moins de 5%.
